# EUROPEAN PATENT APPLICATION

(11) **EP 1 136 079 A1**
(43) Date of publication of application: **26.09.2001**
(21) Application number: 99969956.4
(22) Date of filing: 07.10.1999
(51) Int. Cl.: A61K 45/06

(54) **AGENTS FOR RETARDING CHANGE OF HORMONE-DEPENDENT CANCER INTO HORMONE-INDEPENDENT CANCER**

(30) Priority: 08.10.1998 JP 28679398
(71) Applicant: Takeda Chemical Industries, Ltd., Osaka-shi, Osaka 541-0045 (JP)
(72) Inventor: MATSUTANI, Etsuya, Suita-shi, Osaka 565-0843 (JP); NAITO, Kenichiro, Minoo-shi Osaka 562-0045 (JP)
(74) Representative: Keller, Günter, Dr.
(86) International application number: JP9905533
(87) International publication number: WO0020034

(57) **Abstract**

The present invention relates to a composition that retards the transformation of a hormone-dependent cancer to a non-hormone-dependent cancer, which contains a hormonal agent.

The composition of the present invention that retards the transformation of a hormone-dependent cancer to a non-hormone-dependent cancer, which contains a hormonal agent, is capable of efficiently exhibiting a retarding effect on the transformation of a hormone-dependent cancer to a non-hormone-dependent cancer.

## Description

### FIELD OF THE INVENTION

The present invention relates to a composition that retards the transformation of a hormone-dependent cancer to a non-hormone-dependent cancer, which contains a hormonal agent, and the like.

### BACKGROUND ART

Currently, chemical castration with hormonal agents is used widely for cancer treatment. However, some cancers do not respond to hormonal agents (non-hormone-dependent cancers). In addition, it is known that even a hormone-dependent cancer responsive to hormone therapy can transform to a non-hormone-dependent cancer as a result of proliferation of non-hormone-dependent cancer cells (Laboratory Investigation 67, 540, 1992).

Therefore, in the treatment of cancers (especially prostatic cancer etc.), a therapy effective on both cancers responsive to hormone therapy and cancers not responsive to hormone therapy is ideal; however, no method of effectively treating the two types of cancers is known.

### DISCLOSURE OF THE INVENTION

Through an investigation of changes in the proliferation pathway in cancer cells caused by hormone therapy, the present inventors found a new problem that because the use of a hormonal agent causes non-hormone-dependent cancer cells to proliferate, the anti-cancer therapeutic effect of the hormonal agent does not persist.

The inventors made extensive investigations to resolve this problem and found that the excellent therapeutic effect of a hormonal agent can be maintained and allowed to persist using a composition that retards the transformation of a hormone-dependent cancer to a non-hormone-dependent cancer, which contains the hormonal agent, in therapy using the hormonal agent. The inventors made further investigations based on this finding and developed the present invention.

Accordingly, the present invention provides:
(1) a composition that retards the transformation of a hormone-dependent cancer to a non-hormone-dependent cancer, which contains a hormonal agent,
(2) the composition according to (1) above, wherein said hormonal agent is an LH-RH derivative,
(3) the composition according to (2) above, wherein said LH-RH derivative is an LH-RH agonist,
(4) the composition according to (3) above, wherein said LH-RH derivative is a peptide represented by the formula:

   5-oxo-Pro-His-Trp-Ser-Tyr-Y-Leu-Arg-Pro-Z

   wherein Y represents a residue selected from among DLeu, DAla, DTrp, DSer(tBu), D2Nal and DHis(ImBzl); Z represents NH-C₂H₅ or Gly-NH₂, or a salt thereof,
(5) the composition according to (3) above, wherein said LH-RH derivative is 5-oxo-Pro-His-Trp-Ser-Tyr-DLeu-Leu-Arg-Pro-NH-C₂H₅ or an acetate thereof,
(6) the composition according to (1) above, which contains in addition to a hormonal agent an agent that inhibits the action of a cell growth factor or a receptor thereof,
(7) the composition according to (6) above, wherein said cell growth factor is ① EGF or a substance possessing substantially the same activity as it, ② insulin or a substance possessing substantially the same activity as it or ③ FGF or a substance possessing substantially the same activity as it,
(8) the composition according to (1) above, which is a preventive/therapeutic composition for prostatic cancer, ovarian cancer, cervical cancer or breast cancer,
(9) use of a hormonal agent for retarding the transformation of a hormone-dependent cancer to a non-hormone-dependent cancer,
(10) use of a hormonal agent for producing a pharmaceutical for retarding the transformation of a hormone-dependent cancer to a non-hormone-dependent cancer, and
(11) a method of retarding the transformation of a hormone-dependent cancer to a non-hormone-dependent cancer in a mammal carrying a hormone-dependent cancer, comprising administering a hormonal agent to said mammal.

Furthermore, the present invention provides:
(12) a method of retarding the transformation of a hormone-dependent cancer to a non-hormone-dependent cancer, comprising using a hormonal agent,
(13) a method of retarding the transformation of a hormone-dependent cancer to a non-hormone-dependent cancer, comprising using a hormonal agent and a agent that inhibits the action of a cell growth factor or a receptor thereof,
(14) a method of treating/preventing a cancer, comprising using a hormonal agent to retard the transformation of a hormone-dependent cancer to a non-hormone-dependent cancer,
(15) a method of treating/preventing a cancer, comprising using a hormonal agent and an agent that inhibits the action of a cell growth factor or a receptor thereof to retard the transformation of a hormone-dependent cancer to a non-hormone-dependent cancer,
(16) a method of suppressing the metastasis/recurrence of a cancer, comprising using a hormonal agent to retard the transformation of a hormone-dependent cancer to a non-hormone-dependent cancer,
(17) a method of suppressing the metastasis/recurrence of a cancer, comprising using a hormonal agent and an agent that inhibits the action of a cell growth factor or a receptor thereof to retard the transformation of a hormone-dependent cancer to a non-hormone-dependent cancer,
(18) the method according to (11), (12), (13), (14), (15), (16) or (17) above, wherein said hormonal agent is an LH-RH derivative,
(19) the method according to (18) above, wherein said LH-RH derivative is 5-oxo-Pro-His-Trp-Ser-Tyr-DLeu-Leu-Arg-Pro-NH-C₂H₅ or an acetate thereof,
(20) the method according to (13), (15) or (17) above, wherein said cell growth factor is ① EGF or a substance possessing substantially the same activity as it, ② insulin or a substance possessing substantially the same activity as it or ③ FGF or a substance possessing substantially the same activity as it,
(21) the composition according to (7) above, wherein said cell growth factor is EGF or a substance possessing substantially the same activity as it,
(22) the composition according to (7) above, wherein said cell growth factor is insulin or a substance possessing substantially the same activity as it,
(23) the composition according to (7) above, wherein said cell growth factor is FGF or a substance possessing substantially the same activity as it,
(24) the composition according to (20) above, wherein said cell growth factor is EGF or a substance possessing substantially the same activity as it,
(25) the composition according to (21) above, wherein said cell growth factor is insulin or a substance possessing substantially the same activity as it,
(26) the composition according to (21) above, wherein said cell growth factor is FGF or a substance possessing substantially the same activity as it,
(27) the composition according to (7) or (21) above, wherein said EGF or substance possessing substantially the same activity as it is EGF or heregulin (HER2 ligand),
(28) the composition according to (7) or (22) above, wherein said insulin or substance possessing substantially the same activity as it is insulin, IGF-1 or IGF-2,
(29) the composition according to (7) or (23) above, wherein said FGF or substance possessing substantially the same activity as it is aFGF, bFGF, KGF, HGF or FGF-10,
(30) the composition according to (20) or (24) above, wherein said EGF or substance possessing substantially the same activity as it is EGF or heregulin (HER2 ligand),
(31) the composition according to (20) or (25) above, wherein said insulin or substance possessing substantially the same activity as it is insulin, IGF-1 or IGF-2,
(32) the composition according to (20) or (26) above, wherein said FGF or substance possessing substantially the same activity as it is aFGF, bFGF, KGF, HGF or FGF-10,
(33) the composition according to (6) above, wherein said receptor is an EGF receptor, a heregulin receptor (HER2), insulin receptor-1, insulin receptor-2, IGF receptor, FGF receptor-1 or FGF receptor-2,
(34) the composition according to (13), (15) or (17) above, wherein said receptor is an EGF receptor, a heregulin receptor (HER2), insulin receptor-1, insulin receptor-2, an IGF receptor, FGF receptor-1 or FGF receptor-2,
(35) the composition according to (6) above, wherein said receptor possesses tyrosine kinase activity,
(36) the method according to (13), (15) or (17) above, wherein said receptor possesses tyrosine kinase activity, and
(37) the method according to (9), (10), (11), (12), (13), (14), (15), (16) or (17) above, wherein said cancer is prostatic cancer, ovarian cancer, cervical cancer or breast cancer.

### BRIEF DESCRIPTION OF THE DRAWINGS

Figure 1 is a drawing showing the effect of an androgen antagonist on the expression of the receptor-type tyrosine kinase group gene of a human prostatic cancer cell line.

Figure 2 is a drawing showing the effects of an androgen antagonist and a tyrosine kinase inhibitor on the proliferation of human prostatic cancer cells.

### BEST MODE OF EMBODIMENT OF THE INVENTION

Although any hormonal agent can be used for the present invention, as long as it is pharmacologically useful, preference is given to, for example, hormonal agents having molecular weights of about 300 to about 40,000, preferably about 400 to about 30,000, and more preferably about 500 to about 20,000.

Specifically, there may be mentioned luteinizing hormone-releasing hormone (LH-RH), estrogen preparations, estrogen antagonist preparations (tamoxifen etc.), androgen preparations, androgen antagonist preparations (flutamide, cyproterone acetate), aromatase inhibitors, 5α-reductase inhibitors, lyase inhibitors, insulin, somatostatin, growth hormone, growth hormone-releasing hormone (GH-RH), male hormone-reducing agents, female hormone-reducing agents, prolactin, erythropoietin, adrenocortical hormone, melanocyte-stimulating hormone, thyroid hormone-releasing hormone, thyroid-stimulating hormone, luteinizing hormone, progesterone, follicle-stimulating hormone, vasopressin, oxytocin, calcitonin, gastrin, secretin, pancreozymin, cholecystokinin, angiotensin, human placental lactogen, human chorionic gonadotropin, enkephalin, endorphin, kyotorphin, tuftsin, thymopoietin, thymosin, thymostimulin, thymic humoral factor, blood thymic factor, tumor necrosis factor, colony-stimulating factor, motilin, dynorphin, bombesin, neurotensin, cerulein, bradykinin, atrial natriuretic factor, nerve growth factor, neurotrophic factor, endothelin-antagonistic peptides, derivatives thereof (e.g., agonists, antagonists), and fragments or fragment derivatives thereof.

Said hormonal agent may be a pharmacologically acceptable salt. Such salts include salts with inorganic acids (e.g., hydrochloric acid, sulfuric acid, nitric acid, boric acid), organic acids (e.g., carbonic acid, bicarbonic acid, succinic acid, propionic acid, trifluoroacetic acid), etc., when said hormonal agent has a basic group, such as an amino group.

When said hormonal agent has an acidic group, such as a carboxyl group, such salts include salts with inorganic bases (e.g., alkali metals such as sodium and potassium, alkaline earth metals such as calcium and magnesium), organic bases (e.g., organic amines such as triethylamine, basic amino acids such as arginine), etc. Said hormonal agent may have formed a metal complex compound (e.g., copper complex, zinc complex).

As preferable examples of such hormonal agents, there may be mentioned those which are effective on cancer species with a cell growth factor receptor expressed therein, including LH-RH derivatives which are effective on sex hormone-dependent cancers, such as prostatic cancer, ovarian cancer, cervical cancer and breast cancer.

As specific examples of LH-RH derivatives, there may be mentioned, for example, the peptides described in Treatment with GnRH analogs: Controversies and perspectives [published by The Parthenon Publishing Group Ltd., 1996], Japanese Patent No. 936349, Indication of Existence of Patent No. 503165/1991, Japanese Patent Unexamined Publication Nos. 101695/1991, 97334/1995 and 259460/1996, and elsewhere.

Said LH-RH derivative may be a pharmacologically acceptable salt, such salts including the aforementioned pharmacologically acceptable salts of hormonal agents.

As an LH-RH derivative, there may be mentioned an LH-RH agonist or an LH-RH antagonist. As an LH-RH antagonist, there may be used, for example, a peptide represented by General Formula [I]: X-D2Nal-D4ClPhe-D3Pal-Ser-A-B-Leu-C-Pro-DAla-NH₂ wherein X represents N(4H₂-furoyl)Gly or NAc; A represents a residue selected from among NMeTyr, Tyr, Aph(Atz) and NMeAph(Atz); B represents a residue selected from among DLys(Nic), DCit, DLys(AzaglyNic), DLys(AzaglyFur), DhArg(Et₂), DAph(Atz) and DhCi; C represents Lys(Nisp), Arg or hArg(Et₂), or a salt thereof.

As an LH-RH agonist, there may be used, for example, a peptide represented by General Formula [II]: 5-oxo-Pro-His-Trp-Ser-Tyr-Y-Leu-Arg-Pro-Z wherein Y represents a residue selected from among DLeu, DAla, DTrp, DSer(tBu), D2Nal and DHis(ImBzl); Z represents NH-C₂H₅ or Gly-NH₂, or a salt thereof. A peptide wherein Y is DLeu and Z is NH-C₂H₅ (leuprorelin) or a salt thereof (e.g., acetate) (5-oxo-Pro-His-Trp-Ser-Tyr-DLeu-Leu-Arg-Pro-NH-C₂H₅ or acetate thereof) is particularly preferred.

These peptides can be produced by a method described in the aforementioned literature or patent publications or a modification thereof.

Preferable hormonal agents include estrogen preparations, estrogen antagonist preparations (tamoxifen etc.), androgen preparations, androgen antagonist preparations (flutamide, cyproterone acetate, etc.), aromatase inhibitors, 5α-reductase inhibitors, male hormone-reducing agents, female hormone-reducing agents, and progesterone.

A hormone-dependent cancer refers to a cancer that depends on a hormone, such as prostatic cancer. As preferable targets of administration of the composition of the present invention, there may be mentioned sex hormone-dependent cancers such as prostatic cancer, ovarian cancer, cervical cancer and breast cancer.

A non-hormone-dependent cancer refers to a cancer that does not respond to hormonal agents (e.g., prostatic cancer, ovarian cancer, cervical cancer, breast cancer (especially some cases of prostatic cancer, breast cancer, etc.)), or an above-defined hormone-dependent cancer that has become unresponsive to hormonal agents as a result of a long period of hormone therapy.

The "cancer" in the terms "hormone-dependent cancer" and "non-hormone-dependent cancer" means cancer tissue as a whole, rather than an individual cancer cell.

"To retard the transformation of a hormone-dependent cancer to a non-hormone-dependent cancer (transformation is retarded)" means that in applying hormone therapy to a hormone-dependent cancer described above, the transformation of a hormone-dependent cancer to a non-hormone-dependent cancer is retarded by suppressing or retarding the proliferation of a cancer that has become unresponsive to hormone therapy as a result of a long-term administration of a hormonal agent (non-hormone-dependent cancer).

Specifically, (1) when a hormonal agent described above is used alone, the transformation of a hormone-dependent cancer to a non-hormone-dependent cancer can be retarded by optimizing the combination of its dose, duration of administration, frequency of administration, interval of administration, etc., and (2) when a hormonal agent described above is used in combination with another hormonal agent or an agent that inhibits the action of a cell growth factor or a receptor thereof (it is more preferable to use the aforementioned hormonal agent in combination with a agent that inhibits the action of a cell growth factor or a receptor thereof), the transformation of a hormone-dependent cancer to a non-hormone-dependent cancer can be retarded by optimizing the combination of their doses, durations of administration, frequencies of administration, intervals of administration, etc.

Although any cell growth factor can be used for the present invention, as long as it promotes the growth of cells, it is normally a low-molecular peptide having a molecular weight of not more than 20,000 and exhibits its action at low concentrations by binding to a receptor.

As examples of cell growth factors, there may be mentioned:
① EGF (epidermal growth factor) or a substance possessing substantially the same activity as it (e.g., EGF, heregulin (HER2 ligand)),
② insulin or a substance possessing substantially the same activity as it (e.g., insulin, IGF (insulin-like growth factor)-1, IGF-2),
③ FGF or a substance possessing substantially the same activity as it (e.g., aFGF, bFGF, KGF (keratinocyte growth factor), HGF (hepatocyte growth factor), FGF-10), and
④ other cell growth factors (e.g., CSF (colony-stimulating factor), EPO (erythropoietin), IL-2 (interleukin-2), NGF (nerve growth factor), PDGF (platelet-derived growth factor), TGFβ (transforming growth factor β)).

Said agent that inhibits the action of a cell growth factor or a receptor thereof may be a pharmacologically acceptable salt. Such salts include salts with inorganic acids (e.g., hydrochloric acid, sulfuric acid, nitric acid, boric acid), organic acids (e.g., carbonic acid, bicarbonic acid, succinic acid, propionic acid, trifluoroacetic acid), etc., when said agent that inhibits the action of a cell growth factor or a receptor thereof has a basic group, such as an amino group.

When said agent that inhibits the action of a cell growth factor or a receptor thereof has an acidic group, such as a carboxyl group, such salts include salts with inorganic bases (e.g., alkali metals such as sodium and potassium, alkaline earth metals such as calcium and magnesium), organic bases (e.g., organic amines such as triethylamine, basic amino acids such as arginine), etc. Said agent that inhibits the action of a cell growth factor or a receptor thereof may have formed a metal complex compound (e.g., copper complex, zinc complex).

Although any receptor of a cell growth factor agent can be used for the present invention, as long as it is capable of binding to the aforementioned cell growth factor, there may be mentioned an EGF receptor, a heregulin receptor (HER2), insulin receptor-1, insulin receptor-2, an IGF receptor, FGF receptor-1 or FGF receptor-2.

As agents that inhibit the action of a cell growth factor or a receptor thereof, there may be mentioned, for example, herbimycin and PD153035 (Science 265 (5175), p. 1093 (1994)).

As agents that inhibit the action of a cell growth factor or a receptor thereof, there may also be mentioned HER2 inhibitors. Any HER2 inhibitor can be used for the present invention, as long as it inhibits an activity of HER2 (e.g., phosphorylation activity), whether it is an antibody, a low-molecular compound (synthetic compound, naturally occurring substance), an antisense, a HER2 ligand, heregulin or a partially modified or altered structure thereof. The HER2 inhibitor may also be a substance that indirectly inhibits an HER2 activity by inhibiting an HER2 receptor, like an HER2 receptor antibody.

As low-molecular compounds possessing HER2 inhibiting activity, there may be used, for example, the compounds described in WO98/03505, specifically 1-[3-[4-[2-[(E)-2-phenylethenyl]-4-oxazolylmethoxy]phenyl]propyl]-1,2,4-triazole etc.

When a hormonal agent is used in combination with another hormonal agent or an agent that inhibits the action of a cell growth factor or a receptor thereof, a sufficient retarding effect on the transformation of a hormone-dependent cancer to a non-hormone-dependent cancer can be obtained, even without optimizing the doses, durations of administration, frequencies of administration, intervals of administration, etc. of the individual agents, as long as they fell within ordinary ranges.

It is particularly preferable that the agent of the present invention that inhibits the action of a cell growth factor or a receptor thereof be administered after administration of a hormonal agent, and at a time the effective blood concentration of the hormonal agent has fallen below about 50%, or around a time the receptor of a cell growth factor begins to be expressed upon administration of the hormonal agent.

In addition, by hastening the time of administration initiation as necessary, the retarding effect on the transformation of a hormone-dependent cancer to a non-hormone-dependent cancer is enhanced.

The abbreviations used herein are defined as follows:

| Abbreviation | Designation |
|---|---|
| 5-oxo-Pro | Pyroglutamic acid residue |
| His | Histidine residue |
| Trp | Tryptophan residue |
| Ser | Serine residue |
| Tyr | Tyrosine residue |
| Leu | Leucine residue |
| Arg | Arginine residue |
| Pro | Proline residue |
| DLeu | D-leucine residue |
| DAla | D-alanine residue |
| DTrp | D-tryptophan residue |
| N(4H₂-furoyl)Gly | N-tetrahydrofuroylglycine residue |
| NAc | N-acetyl group |
| D2Nal | D-3-(2-naphthyl)alanine residue |
| D4ClPhe | D-3-(4-chloro)phenylalanine residue |
| D3Pal | D-3-(3-pyridyl)alanine residue |
| NMeTyr | N-methyltyrosine residue |
| Aph(Atz) | N-[5'-(3'-amino-1'H-1',2',4'-triazolyl)]phenylalanine residue |
| NMeAph(Atz) | N-methyl-[5'-(3'-amino-1'H-1',2',4'-triazolyl)]phenylalanine residue |
| DLys(Nic) | D-(e-N-nicotinoyl)lysine residue |
| Dcit | D-citrulline residue |
| DLys(AzaglyNic) | D-(azaglycylnicotinoyl)lysine residue |
| DLys(AzaglyFur) | D-(azaglycylfuranyl)lysine residue |
| DhArg(Et₂) | D-(N,N'-diethyl)homoarginine residue |
| DAph(Atz) | D-N-[5'-(3'-amino-1'H-1',2',4'-triazolyl)]phenylalanine residue |
| DhCi | D-homocitrulline residue |
| Lys(Nisp) | (e-N-isopropyl)lysine residue |
| hArg(Et₂) | (N,N'-diethyl)homoarginine residue |
| DSer(tBu) | D-O-(t-butyl)serine residue |
| Dhis(ImBzl) | N'-butylhistidine residue |

Abbreviations for other amino acids used in the present specification are based on abbreviations specified by the IUPAC-IUB Commission on Biochemical Nomenclature (European Journal of Biochemistry, Vol. 138, pp. 9-37 (1984)) or abbreviations in common use in relevant fields. When an optical isomer may be present in amino acid, it is of the L-configuration, unless otherwise stated.

The composition of the present invention is of low toxicity and can be used as a pharmaceutical composition as-is, or in a mixture with a commonly known pharmaceutically acceptable carrier etc. in mammals (e.g., horses, bovines, dogs, cats, rats, mice, rabbits, pigs, monkeys).

As a hormonal agent and if necessary an agent that inhibits the action of a cell growth factor or a receptor thereof, formulated with a pharmaceutically acceptable carrier, the composition of the present invention can be administered orally or non-orally in the form of solid preparations such as tablets, capsules (including soft capsules and microcapsules), granules, powders, and suppositories; or liquid preparations such as syrups and injections. Alternatively, a hormonal agent and an agent that inhibits the action of a cell growth factor or a receptor thereof can also be prepared as separate preparations.

As pharmaceutically acceptable carriers, there may be used various organic or inorganic carrier substances in common use for pharmaceutical preparations, including excipients, lubricants, binders, and disintegrating agents in solid preparations; and solvents, dissolution aids, suspending agents, isotonizing agents, buffers, and soothing agents in liquid preparations. Such pharmaceutical additives as antiseptics, antioxidants, coloring agents, and sweetening agents can also be used as necessary.

As examples of preferable excipients, there may be mentioned, for example, lactose, sucrose, D-mannitol, starch, crystalline cellulose, and light silicic anhydride.

As examples of preferable lubricants, there may be mentioned, for example, magnesium stearate, calcium stearate, talc, and colloidal silica.

As examples of preferable binders, there may be mentioned, for example, crystalline cellulose, sucrose, D-mannitol, dextrin, hydroxypropyl cellulose, hydroxypropylmethyl cellulose, and polyvinylpyrrolidone.

As examples of preferable disintegrating agents, there may be mentioned, for example, starch, carboxymethyl cellulose, carboxymethyl cellulose calcium, crosslinked carmellose sodium, and carboxymethyl starch sodium.

As examples of preferable solvents, there may be mentioned, for example, water for injection, alcohol, propylene glycol, macrogol, sesame oil, and corn oil.

As examples of preferable dissolution aids, there may be mentioned, for example, polyethylene glycol, propylene glycol, D-mannitol, benzyl benzoate, ethanol, trisaminomethane, cholesterol, triethanolamine, sodium carbonate, and sodium citrate.

As examples of preferable suspending agents, there may be mentioned, for example, surfactants such as stearyltriethanolamine, sodium lauryl sulfate, laurylaminopropionic acid, lecithin, benzalkonium chloride, benzethonium chloride, and monostearic glycerol; and hydrophilic polymers such as polyvinyl alcohol, polyvinylpyrrolidone, carboxymethyl cellulose sodium, methyl cellulose, hydroxymethyl cellulose, hydroxyethyl cellulose, and hydroxypropyl cellulose.

As examples of preferable isotonizing agents, there may be mentioned, for example, sodium chloride, glycerol, and D-mannitol.

As examples of preferable buffers, there may be mentioned, for example, buffer solutions of phosphates, acetates, carbonates, citrates, etc.

As examples of preferable soothing agents, there may be mentioned, for example, benzyl alcohol.

As examples of preferable antiseptics, there may be mentioned, for example, para-oxybenzoic acid esters, chlorobutanol, benzyl alcohol, phenethyl alcohol, dehydroacetic acid, and sorbic acid.

As examples of preferable antioxidants, there may be mentioned, for example, sulfites and ascorbic acid.

The content of a hormonal agent in the agent of the present invention is normally about 0.1 to 30% (w/w), preferably about 1 to 20% (w/w), and more preferably about 5 to 10% (w/w), to the total amount of the preparation, although it varies depending on dosage form, method of administration, carrier, etc.

The content of an agent that inhibits a cell growth factor or a receptor thereof (especially a low-molecular compound) in the composition of the present invention is normally about 0.1 to 90% (w/w) to the total amount of the preparation, although it varies depending on dosage form, method of administration, carrier, etc.

The content of each pharmaceutical additive is normally about 0.1 to 99.9% (w/w), preferably about 10 to 99.9% (w/w), and more preferably about 20 to 90% (w/w), to the total amount of the preparation.

Although the dose of the composition of the present invention varies depending on the kind of the hormonal agent, the kind of the agent that inhibits the action of a cell growth factor or a receptor thereof, route of administration, symptoms, etc., it is preferably about 1.0 to 100 mg/kg body weight, more preferably about 5.0 to 50 mg/kg body weight, per day, based on a compound, for example, when an LH-RH derivative is administered subcutaneously as an anticancer composition to a patient with breast cancer or prostatic cancer (body weight 40 to 80 kg) and the agent that inhibits the action of a cell growth factor or a receptor thereof is a low-molecular compound. This amount can be administered once or in 2 to 3 divided portions per day. The dose of the LH-RH derivative is preferably about 1.0 to 100 mg/kg body weight, more preferably about 1.0 to 50 mg/kg body weight, per day, based on a compound, for example, when it is administered to a patient with breast cancer or prostatic cancer (body weight 40 to 80 kg). This amount can be administered once or in 2 to 3 divided portions per day. When the agent that inhibits the action of a cell growth factor or a receptor thereof is an antibody, it can be administered intravenously, subcutaneously, locally (tumor), or the like, on consecutive days or intermittently, at a dose of normally about 1 to 2,000 mg/kg/week, preferably about 5 to 1,000 mg/kg/week.

The composition of the present invention is capable of maintaining or allowing to persisting the pharmacologic action of a hormonal agent normally by about 1.5 times or more, more specifically by about 1.5 to 3 times.

Furthermore, the composition of the present invention can be formulated with another active ingredient, e.g., a chemotherapeutic composition or an immunotherapeutic composition.

As chemotherapeutic agents, there may be mentioned, for example, alkylating agents (e.g., cyclophosphamide, ifosfamide), metabolism antagonists (e.g., methotrexate, 5-fluorouracil), anticancer antibiotics (e.g., mitomycin, adriamycin), plant-derived anticancer agents (e.g., vincristine, vindesine, taxol), cisplatin, carboplatin, and etoposide.

As immunotherapeutic agents, there may be mentioned, for example, microbial or fungal components (e.g., muramyl dipeptide derivatives, picibanil), polysaccharides possessing immunopotentiating activity (e.g., lentinan, sizofiran, krestin), and cytokines obtained by gene engineering techniques (e.g., interferons, interleukins).

### Examples

The present invention is hereinafter described in more detail by means of, but is not limited to, the following working examples, reference examples and experimental examples.

### Reference Example 1

To a suspension of aluminum lithium hydride (350 mg) in diethyl ether (10 ml), a solution of ethyl 4-[4-[2-[(E)-2-phenylethenyl]-4-oxazolylmethoxy]phenyl]butyrate (3.00 g) in diethyl ether (10 ml)-tetrahydrofuran (10 ml) was added drop by drop at 0°C. After stirring at 0°C for 1 hour and at room temperature for 1 hour, water was added; the reaction mixture was acidified with 2 N hydrochloric acid and extracted with ethyl acetate. After the ethyl acetate layer was washed with water and dried (MgSO₄), the solvent was distilled off. The residue was subjected to silica gel column chromatography and eluted with ethyl acetate-hexane (1:1, v/v) to yield 4-[4-[2-[(E)-2-phenylethenyl]-4-oxazolylmethoxy]phenyl]butanol (2.00 g, 74%), which was recrystallized from ethyl acetate-hexane to yield a colorless needle crystal having a melting point of 90 to 91°C.

### Reference Example 2

In the same manner as in Reference Example 1, ethyl 3-[4-[2-[(E)-2-phenylethenyl]-4-oxazolylmethoxy]phenyl]propionate was reduced to yield 3-[4-[2-[(E)-2-phenylethenyl]-4-oxazolylmethoxy]phenyl]propanol (recovery 94%), which was recrystallized from ethyl acetate-hexane to yield a colorless needle crystal having a melting point of 95 to 96°C.

### Reference Example 3

To a solution of 3-[4-[2-[(E)-2-phenylethenyl]-4-oxazolylmethoxy]phenyl]propanol as obtained in Reference Example 2 (760 mg), tributylphosphine (1.01 g) and 1,2,4-triazole (280 mg) in tetrahydrofuran (15 ml), diethyl azodicarboxylate (700 mg) was added drop by drop at 0°C. After refluxing under heating for 1 hour, the reaction mixture was poured over water and extracted with ethyl acetate. After the ethyl acetate layer was washed with water and dried (MgSO₄), the solvent was distilled off. The residue was subjected to silica gel column chromatography and eluted with ethyl acetate-hexane (2:1, v/v) to yield a crystal, which was recrystallized from ethyl acetate-hexane to yield 1-[3-[4-[2-[(E)-2-phenylethenyl]-4-oxazolylmethoxy]phenyl]propyl]-1,2,4-triazole (540 mg, 70%) as a colorless prismatic crystal having a melting point of 108 to 109°C.

| Reference Example 4 | |
|---|---|
| (1) Compound of Reference Example 3 | 10.0 mg |
| (2) Lactose | 60.0 mg |
| (3) Corn starch | 35.0 mg |
| (4) Gelatin | 3.0 mg |
| (5) Magnesium stearate | 2.0 mg |

These ingredients were mixed and tabletted.

### Example 1

Tablets that were obtained in Reference Example 4 and a sustained-release preparation obtained by the method described below were used in combination.

400 mg of leuprorelin acetate (produced by Takeda Chemical Industries, Ltd.) was dissolved in 0.5 ml of distilled water to yield an aqueous phase solution. This solution was added to a solution of 4 g of poly-DL-lactic acid [Lot No. 870818; weight-average molecular weight 18000 (microcapsule Lot Nos. 244 and 245) and Lot No. 880622; weight-average molecular weight 18200, degree of dispersion 1.76 (microcapsule Lot No. 248)] in 7.5 ml of dichloromethane and emulsified for about 60 seconds using a compact homogenizer (Polytron, produced by Kinematica, Switzerland) to yield a W/O emulsion. This emulsion was cooled to 15°C, injected into 1,000 ml of a 0.25% aqueous solution of polyvinyl alcohol, previously cooled to 15°C, and treated using a compact homogenizer to yield a W/O/W emulsion. While the W/O/W emulsion was stirred, the dichloromethane was volatilized to solidify the inner W/O emulsion, which was then collected using a centrifuge.

This was dispersed again in distilled water and centrifuged; the agent, dispersing composition, etc. released were washed.

The microcapsules collected were lyophilized to achieve complete solvent removal and dehydration and to yield a powder.

### Experimental Example 1

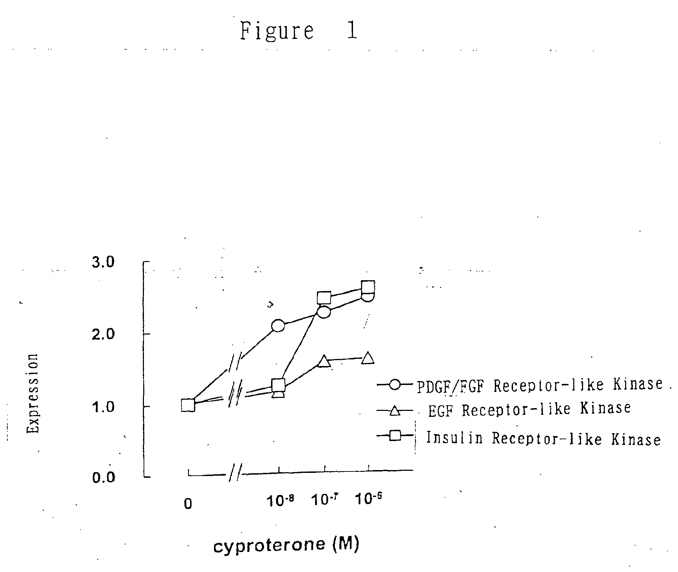

5 ml of a cell suspension (2 x 10⁵ cells/ml) of the human prostatic cancer cell line LNCaP (ATCC (American Type Culture Collection) Catalog No. CRL1740, J.S. Horoszewicz, Cancer Res. 43: 1809-1818 (1983)) was dispensed to 60 mm Petri dishes and cultured at 37°C in the presence of 5% CO₂ overnight. The medium was replaced with a medium containing a preset concentration of cyproterone acetate (Sigma; Cat. No. C-3412) and cultivation was continued at 37°C in the presence of 5$ CO₂ for 3 days. After the medium was removed, RNA was extracted from each Petri dish by the acidic guanidine isocyanate/phenol/chloroform method. With these RNA extracts as templates, complementary DNAs were synthesized using an oligo-dT adapter primer (Takara Shuzo) as a primer, to yield PCR reaction templates. Primers specific for the receptor type tyrosine kinase group were synthesized on the basis of sequences common to the kinase regions, i.e., HisArgAspLeuAlaAla and SerAspValTrpSer (Hanks et al., 1988). Specifically, 5'-CA(C/T)(C/A)GGGA(C/T)(C/T)TGGC (A/T/C)GC (sense primer) and 5'-A(A/G)CTCCA(A/C)AC(A/G)TC(A/G)CT (antisense primer) were synthesized for EGF receptor-like kinase, 5'-CA(C/T)(C/A)G(G/A)GAC(C/T)T(G/T)GC(A/T)GC (sense primer) and 5'-A(A/G)CTCCA(A/C)ACGTC(A/C)GA (antisense primer) for insulin receptor-like kinase, and 5'-CA(C/T)(C/A)G(G/A)GAC(C/T)TGGC(A/G)GC (sense primer) and 5'-A(A/G)GACCA(G/C)AC(A/G)TC(A/G)CT (antisense primer) for PDGF receptor-like kinase and FGF receptor-like kinase. An amplification reaction was carried out in 35 cycles, each amplification cycle comprising 95°C × 1 min (denaturation), 40°C × 1 min (annealing), and 72°C × 1 min (synthesis). The amount of each receptor kinase group expressed was quantified by analyzing an image obtained by staining the PCR reaction product with ethidium bromide after agarose gel electrophoresis (4%) (Figure 1). For the purpose of standardization, each amount of expression was obtained as the ratio to the amount of β-actin expressed in the same sample. The numerical figures in Figure 1 indicate the amounts of expression at the various time points with the amount of expression in the absence of cyproterone acetate taken as 1.

β-actin was amplified in 25 cycles of amplification (95°C × 0.5 min, 60°C × 1 min, 72°C × 0.5 min) using 5'-ATCTGGCACCACACCTTCTACAATGAGCTGCG (sense) and 5'-CGTCATACTCCTGCTTGCTGATCCACATCTGC (antisense) as primers.

### Experimental Example 2

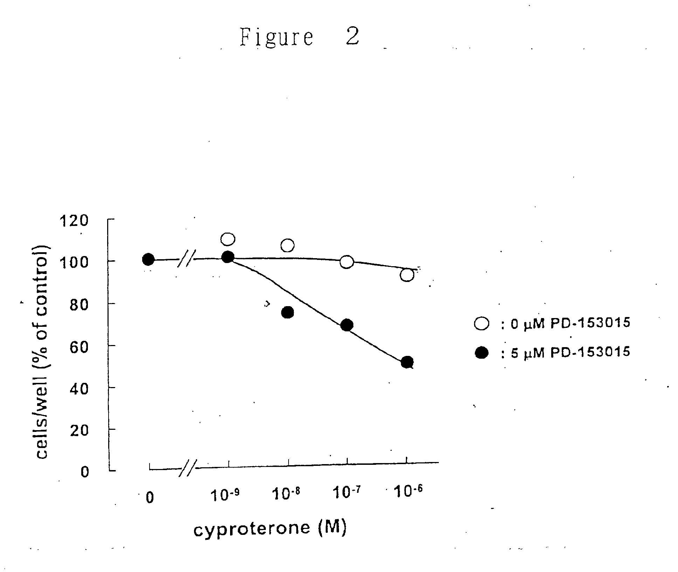

Cells of the human prostatic cancer cell line LNCaP in passage culture were trypsinized and suspended in RPMI1640 medium (Gibco BRL) containing 10% fetal bovine serum (BioWhittaker). The cell density of this cell suspension was determined using a Coulter counter and adjusted to 2 × 10⁴ cells/ml using the medium described above. This suspension was dispensed to a 24-well multi-well culture plate (Becton Dickinson) at 0.5 ml per well and cultured at 37°C in the presence of 5% CO₂ overnight. To each well, 0.5 ml of a medium . containing a preset concentration of 2 × 10⁻⁹ to 2 × 10⁻⁶ M cyproterone acetate (Sigma; Cat. No. C-3412) or a medium containing a preset concentration of cyproterone acetate and 10 mM PD153035 (Science 265 (5175), p. 1093 (1994); Japan Society for Bioscience, Biotechnology and Agrochemistry 1996 Annual Meeting Abstract No. 21a14 (March 30, 1996, Kyoto)). After cultivation at 37°C in the presence of 5% CO₂ for 4 days, the number of cells in each well was determined using a Coulter counter (Figure 2).

### INDUSTRIAL APPLICABILITY

The composition of the present invention that retards transformation of a hormone-dependent cancer to a non-hormone-dependent cancer, which contains a hormonal agent, is capable of efficiently exhibiting a retarding effect on the transformation of a hormone-dependent cancer to a non-hormone-dependent cancer.

## Claims

1. A composition that retards the transformation of a hormone-dependent cancer to a non-hormone-dependent cancer, which contains a hormonal agent.

2. The composition as claimed in claim 1, wherein said hormonal agent is an LH-RH derivative.

3. The composition as claimed in claim 2, wherein said LH-RH derivative is an LH-RH agonist.

4. The composition as claimed in claim 3, wherein said LH-RH derivative is a peptide represented by the formula:
5-oxo-Pro-His-Trp-Ser-Tyr-Y-Leu-Arg-Pro-Z
wherein Y represents a residue selected from among DLeu, DAla, DTrp, DSer(tBu), D2Nal and DHis(ImBzl); Z represents NH-C₂H₅ or Gly-NH₂, or a salt thereof.

5. The composition as claimed in claim 3, wherein said LH-RH derivative is 5-oxo-Pro-His-Trp-Ser-Tyr-DLeu-Leu-Arg-Pro-NH-C₂H₅ or an acetate thereof.

6. The composition as claimed in claim 1, which contains in addition to a hormonal agent an agent that inhibits the action of a cell growth factor or a receptor thereof.

7. The composition as claimed in claim 6, wherein said cell growth factor is ① EGF or a substance possessing substantially the same activity as it, ② insulin or a substance possessing substantially the same activity as it, or ③ FGF or a substance possessing substantially the same activity as it.

8. The composition as claimed in claim 1, which is a preventive/therapeutic composition for prostatic cancer, ovarian cancer, cervical cancer or breast cancer.

9. Use of a hormonal agent for retarding the transformation of a hormone-dependent cancer to a non-hormone-dependent cancer.

10. Use of a hormonal agent for producing a pharmaceutical for retarding the transformation of a hormone-dependent cancer to a non-hormone-dependent cancer.

11. A method of retarding the transformation of a hormone-dependent cancer to a non-hormone-dependent cancer in a mammal carrying a hormone-dependent cancer, comprising administering a hormonal agent to said mammal.
